# EUROPEAN PATENT SPECIFICATION

(11) **EP 3 131 641 B1**
(45) Date of publication and mention of the grant of the patent: **24.07.2019**
(21) Application number: 15716504.4
(22) Date of filing: 13.04.2015
(51) Int. Cl.: A61Q 11/00, A61K 8/02, A61K 8/19, A61K 8/73

(54) **SOLID ORAL CARE COMPOSITIONS COMPRISING CALCIUM CARBONATE AND CARRAGEENAN**
FESTE MUNDPFLEGEZUSAMMENSETZUNGEN ENTHALTEND CALCIUMCARBONAT UND CARRAGEENAN
COMPOSITIONS DE SOIN ORAL SOLIDE COMPRENANT DU CARBONATE DE CALCIUM ET CARRAGHÉNANE

(30) Priority: 17.04.2014 EP 14165049
(43) Date of publication of application: 22.02.2017
(73) Proprietor: Unilever PLC, London Greater London EC4Y 0DY (GB); Unilever N.V., 3013 AL Rotterdam (NL)
(72) Inventor: ASHCROFT, Alexander Thomas, Wirral Merseyside CH63 3JW (GB); WILSON, William John, Wirral Merseyside CH63 3JW (GB)
(74) Representative: Tansley, Sally Elizabeth
(86) International application number: PCT/EP2015/057922
(87) International publication number: WO 2015/158639

(56) References cited:
- WO-A2-2010/115037
- GB-A- 2 163 348
- DATABASE GNPD [Online] MINTEL; August 2011 (2011-08), Archtek: "Toothpaste tablets", XP002731023, Database accession no. 1590507

## Description

### Field of the Invention

The present invention is concerned with solid oral care compositions.

### Background of the Invention

Oral care tablets are forms of dentifrice formulations that use less water when cleaning the teeth and so conserve water.

Toothpaste tablets containing calcium carbonate have been used commercially such as those sold as Archteck "toothpaste tablets" (GNPD database August 20011).

US 3,431,339 describes a compressed dental tablet for use in place of toothpaste. The tablet contains a blend of a water-soluble anticaries agent, polishing agent, foaming agent and releasable matrix, all in a substantially anhydrous form. Advantages of the format are stated to be improved storage stability of the anticaries agent in the dry tablet and improved cleaning of the lingual surfaces, compared to conventional toothpaste.

US 3,116,208 discloses a dental cleanser in tablet form comprised of calcium carbonate and sodium lauryl sulphate. The tablet may be crushed by the teeth and the sodium lauryl sulphate causes foaming upon brushing the teeth.

US2007/0154409 describes a tooth cleansing tablet consisting of a quantity of tooth powder formed into a firm tablet. The tablet is made from a combination of a mild tooth abrasive with a binding agent and flavouring. The tablet is protected by a coating which provides water resistance and can be made of any ingredient commonly used to form the coatings on candies or gum balls.

US2004/0101494 discloses chewable compressed oral care tablets comprising water soluble gums. The tablets are said to deliver oral care actives directly into the tooth surface.

It is an object of the present invention to provide tablets with improved fresh feel to the mouth and which quickly break down.

### Summary of the Invention

The invention relates to the use of carrageenan in an oral care tablet to enhance the breakdown time of the tablet in the mouth.

Also described is the use of carrageenan in an oral care tablet to enhance breath freshness.

### Detailed Description of the Invention

The present invention relates to solid oral care composition, preferably the composition is in the form of a tablet, more preferably in the form a compressed tablet.

Composition of the invention comprise calcium carbonate, the calcium carbonate being a particulate material which acts to mechanically remove debris from a tooth surface and debride plaque.

Suitable particulate calcium carbonates may be characterised by the specific shape and size of their constituent primary particles.

By "primary particles" is meant individual particles, defined as the smallest discrete particles that can be seen by electron microscopy analysis (such as, for example, individual crystals).

The primary particles may associate under certain conditions to form larger secondary structures such as aggregates or agglomerates.

For the purposes of the present invention, a suitable source of particulate calcium carbonate includes crystalline calcium carbonates.

The term "crystalline" (in the context of particulate calcium carbonate) generally means a particulate calcium carbonate in which at least 50% by weight, preferably at least 75% by weight, more preferably at least 90% by weight, most preferably more than 95% by weight and ideally more than 99% by weight of the calcium carbonate particles are in the form of crystals.

The term "crystal" means an essentially fully dense solid composed of atoms arranged in an orderly repetitive array bounded by plane surfaces which are the external expression of internal structure. Crystals may be identified and characterised by standard techniques known to those skilled in the art such as X-ray diffraction.

Crystalline calcium carbonates suitable for use in the invention are available naturally (through the extraction and processing of natural ores) or synthetically (through chemical precipitation). There are three main crystalline polymorphs: calcite, aragonite, and less commonly found, vaterite. The vaterite form of calcium carbonate is metastable and irreversibly transforms into calcite and aragonite. There are many different morphologies(crystal habits) for each of these crystalline forms. The calcite crystalline polymorph is the most commonly used crystal form of calcium carbonate. Over 300 crystalline forms of calcite have been reported in the literature.

Particulate calcium carbonates suitable for use in the invention (such as the materials described above) have an average particle size d(50) which can vary over a wide range. Usually the average particle size d(50) is 50 microns or less.

Particle (e.g. crystal) size may be determined by standard techniques known to those skilled in the art such as sedimentation. For the present invention the particle size should be determined using a Malvern mastersizer
Good cleaning performance has been obtained with crystalline calcium carbonates in which at least 50% by weight, preferably at least 80% by weight of the crystals are rhombohedral calcite crystals with an average particle size d(50) of 30 microns or less, preferably15 microns or less. Most preferable are crystalline calcium carbonates with an average particle size from 0.1 to 30 microns, preferably 0.1 to 15 microns, most preferably 2 to 10 microns (measured using a Malvern mastersizer).

Suitable sources of crystalline calcium carbonate of the size and shape defined above include natural ground calcium carbonate abrasives, generally obtainable from mining and mechanical grinding of sedimentary rocks such as limestone or chalk, or metamorphic rocks such as marble. Natural calcium carbonate is usually of the calcitic polymorph, with a crystal morphology which may typically be characterised as rhombohedral.

Preferred natural ground calcium carbonate abrasives of the type defined above are selected from ground limestone, chalk or marble, optionally refined or partially refined to remove impurities, and having an average particle size ("median ESD" as described above d(50)) of about 30 microns or less, more preferably ranging from about 1 to 15 microns, and most preferably ranging from about 2 to 10 microns. Commercially available examples include those materials sold by the company OMYA™ AG under the names OMYACARB™ 2-AV and OMYACARB™ 5-AV. These are fine ground high purity white marble abrasives having an average particle size ("median ESD" as described above) of about 2 and about 5 microns respectively.

Other types of particulate calcium carbonate may also be suitable, depending on the particular balance of cleaning and abrasion required from a consumer perspective.

Mixtures of any of the above described abrasive cleaning agents may also be used.

The total amount of calcium carbonate in the composition of the invention will depend on the particular agent (or agents) used, but generally ranges from 3 to 80%, preferably from 15% to 75%, more preferably from 30% to 70% most preferably from 40 to 50 wt% by weight by total weight of the composition.

Compositions of the invention comprise carrageenan, preferably kappa carrageenan.

The weight of carrageenan in the composition is preferably from 0.1 to 3 wt%, more preferably from 0.5 to 2 wt% of the total composition.

It is preferable if the polyethylene glycol is present as part of a mixture within the tablet rather than an external coating.

The weight ratio of carrageenan to calcium carbonate is from 1:20 to 1:100, more preferably from 1:30 to 1:50.

Suitable foaming agents for use in forming the composition include surfactants. Surfactants help to increase the rate of dissolution of the solid oral care composition when in contact with saliva, and assist in foaming of the composition during usage.

Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines.

Preferred surfactants for use in the invention include those anhydrous surfactants selected from sodium lauryl sulfate, sodium lauryl sulfoacetate, cocamidopropyl betaine, sodium alpha olefin sulfonate, dioctyl sodium sulfosuccinate, sodium dodecyl benzene sulfonate and mixtures thereof.

Mixtures of any of the above described materials may also be used.

The total amount of surfactant incorporated into the composition of the invention generally ranges from about 0.2 to about 5%, by total weight surfactant based on the total weight of the composition.

The composition may also include various additional ingredients to improve aspects such as ease of processing, product performance and/or consumer acceptability.

For example, the composition may include effervescent agents which promote foaming by the provision of bubbles. Suitable effervescent agents for use in this context include acid/carbonate salt couples which provide effervescence by the reaction of the carbonate salt with the acid. Sodium bicarbonate and sodium carbonate represent preferred carbonate salts. However potassium, ammonium, magnesium, calcium carbonate or other metal or organic salts can also be used. Suitable acids include citric acid, fumaric acid, tartaric acid, malic acid, adipic acid and other edible acids.

The amount of effervescent agent incorporated into the composition of the composition of the invention generally ranges from about 0.5 to about 10%, preferably from about 1 to 7%, by total weight effervescent agent (e.g. acid/carbonate salt couple) based on the total weight of the composition.

Mixtures of any of the above described materials may also be used.

The composition will preferably include one or more organic polyols having 2 or more hydroxyl groups in the molecule (hereinafter termed "organic polyols"). These materials may serve as binders to aid in compression of the composition during processing. They may also act as humectants. Humectants help to keep the composition from hardening or crystallizing upon exposure to air. They also help to give the composition a moist feel to the mouth, and may in some cases impart a desirable sweetness. Suitable organic polyols for use in this context include sucrose, dextrose, maltose, fructose, glycerol, sorbitol, xylitol, mannitol, lactitol, maltitol, isomalt, erythritol, polyethylene glycol, polypropylene glycol, propylene glycol, and hydrogenated partially hydrolyzed polysaccharides such as hydrogenated starch hydrolysates. Preferred organic polyols for use in this context are non-cariogenic polyhydric alcohols such as glycerol, sorbitol, maltitol and xylitol.

Mixtures of any of the above described materials may also be used.

The amount of organic polyol incorporated into the composition of the composition of the invention generally ranges from about 5 to about 70%, preferably from about 10 to 40%, by total weight organic polyol based on the total weight of the composition.

The composition of the composition of the invention is typically non-aqueous. By "non-aqueous" it is generally meant that water is not deliberately added to the composition in any significant quantity. However, the term "non-aqueous" does not mean that small amounts of water cannot be present, for example as a consequence of its association with hygroscopic raw materials. Accordingly, for the purposes of this invention, the term "non-aqueous" generally means that water is present in an amount no greater than about 5%, more preferably no greater than about 3% by weight based on the total weight of the composition.

The solid oral care composition of the invention will generally take the form of a discrete, single unit dose such as a pellet, pastille or tablet. Such a single unit dose will typically range in size from 200 mg to 5000 mg, preferably from 250 mg to 2000 mg, more preferably from 500 to 1500 mg.

Tablets of the invention are preferably manufactured using a compaction process to 1 to 8 tons. Preferably the tablets are not coated.

Compositions according to the invention are particularly suitable as a vehicle for oral care actives which may be physically or chemically incompatible with water, or which may function less efficiently in an aqueous environment.

Specific examples of oral care actives which may be particularly suitable for inclusion in the compositions of the invention are:
water-soluble or sparingly water-soluble sources of zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate;
oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8-hydroxyquinoline derivatives), lactoperoxidase, lactoferrin, lysozyme and mixtures thereof;
fluoride sources such as sodium fluoride, stannous fluoride, sodium monofluorophosphate, zinc ammonium fluoride, tin ammonium fluoride, calcium fluoride, cobalt ammonium fluoride and mixtures thereof;
plant-derivable antioxidants such as flavonoid, catechin, polyphenol, and tannin compounds and mixtures thereof;
antioxidant vitamins such as tocopherols and/or derivatives thereof, ascorbic acid and/or derivatives thereof and mixtures thereof.
agents for the remineralisation of teeth (i.e the *in situ* generation of hydroxyapatite on teeth), such as a mixture of a calcium source and a phosphate source which, when delivered to the teeth results in the *in situ* generation of hydroxyapatite on teeth.

Mixtures of any of the above described materials may also be used.

Compositions of the present invention may also contain further optional ingredients customary in the art such as anticalculus agents, buffers, flavouring agents, sweetening agents, colouring agents, opacifying agents, preservatives, antisensitivity agents, antimicrobial agents and the like.

The solid oral care composition of the invention is used to clean the surfaces of the oral cavity. The composition is an oral composition that is not intentionally swallowed for purposes of systemic administration of therapeutic agents, but is applied to the oral cavity, used to treat the oral cavity and then expectorated. Typically the composition is used in conjunction with a cleaning implement such as a toothbrush, usually by applying it to the bristles of the toothbrush and then brushing the accessible surfaces of the oral cavity.

The invention is further illustrated with reference to the following, non-limiting Examples. Examples of the invention are illustrated by a number, comparative Examples are illustrated by a letter.

### EXAMPLE

| Tradename | Chemical Name | Example A | Example 1 |
|---|---|---|---|
| | | Wt% | Wt% |
| Empicol LZN | Sodium lauryl sulphate | 3 | 3 |
| Menthol | Menthol | 0.5 | 0.5 |
| Novamint | Synthetic flavour | 6 | 6 |
| Chalk | Calcium carbonate | 45.2 | 45.2 |
| Sodium saccharin | Sodium saccharin | 0.3 | 0.3 |
| Xylisorb DC | Xylitol | To 100 | To 100 |
| | Kappa Carrageenan | 0 | 1 |

To form the tablet the materials were blended together. Once blended a 1g amount is weighed into a tablet die and compressed to the desired force (3 ton).

The tablet was assessed by a trained panel. All tablets were presented in petri dishes. The tablets were placed in the mouth and chewed, then when foam developed; panellists brushed their teeth and eventually spit out any residue then rinsed if needed. The panel 'rated' the intensity of the smooth feeling attribute.

In-between prototypes participants were asked to eat 2-3 crisps (Walker ready salted) and rinse their mouth with room-temperature water. This process cleanses the palate of previous tastes. After the last prototype, only rinsing if required.

The results of the panel test were as follows

| Sensory Attribute | Score Example 1 | Score Example A |
|---|---|---|
| Freshness immediately after use | 78.3 | 77.3 |
| Speed of break down | 79.4 | 77 |

The higher the score the more intense the attribute.

The results show that tablets of the invention feel fresh in the mouth and break down quickly.

## Claims

1. Use of carrageenan in an oral care tablet to enhance the breakdown time of the tablet in the mouth.

2. Use according to claim 1 in which the tablet is a solid oral care compressed tablet , the composition of the tablet comprising a mixture of calcium carbonate and carrageenan, in which the weight ratio of carrageenan to calcium carbonate is from 1:20 to 1:100.

3. Use according to claim 1 or claim 2 in which the carrageenan is kappa carrageenan.

4. Use according to claim 2 or claim 3 in which the calcium carbonate has a particle size d(50)ranging from 0.1 to 30 microns, preferably 0.1 to 15 microns, most preferably 2 to 10 microns as measured by Malvern mastersizer.

5. Use according to any preceding claim in which the tablet further comprises a foaming agent selected from the group consisting of anhydrous surfactants selected from sodium lauryl sulfate, sodium lauryl sulfoacetate, cocamidopropyl betaine, sodium alpha olefin sulfonate, dioctyl sodium sulfosuccinate, sodium dodecyl benzene sulfonate and mixtures thereof.

6. Use according to any preceding claim, in which the composition also includes one or more organic polyols selected from glycerol, sorbitol, maltitol, xylitol and mixtures thereof.

7. Use according to any preceding claim in which the level of carrageenan is from 0.1 to 3 wt% of the total composition.

## Patentansprüche

1. Verwendung von Carrageen in einer Mundpflegetablette, um die Abbauzeit der Tablette in dem Mund zu verbessern.

2. Verwendung nach Anspruch 1, in welcher die Tablette eine feste Mundpflegepresstablette ist, wobei die Zusammensetzung der Tablette eine Mischung von Calciumcarbonat und Carrageen umfasst, in welcher das Gewichtsverhältnis von Carrageen zu Calciumcarbonat von 1:20 bis 1:100 beträgt.

3. Verwendung nach Anspruch 1 oder Anspruch 2, wobei das Carrageen Kappa-Carrageen ist.

4. Verwendung nach Anspruch 2 oder Anspruch 3, wobei das Calciumcarbonat eine zwischen 0,1 und 30 Mikron liegende Teilchengröße d(50) aufweist, vorzugsweise zwischen 0,1 und 15 Mikron, höchst bevorzugt zwischen 2 und 10 Mikron, gemessen mittels Malvern Mastersizer.

5. Verwendung nach irgendeinem vorhergehenden Anspruch, wobei die Tablette ferner ein Schäumungsmittel enthält, ausgewählt aus der Gruppe, die aus wasserfreien Tensiden, ausgewählt aus Natriumlaurylsulfat, Natriumlaurylsulfoacetat, Cocamidopropylbetain, Natrium-alpha-olefinsulfonat, Dioctylnatriumsulfosuccinat, Natriumdodecylbenzolsulfonat und Mischungen davon, besteht.

6. Verwendung nach irgendeinem vorhergehenden Anspruch, wobei die Zusammensetzung auch ein oder mehrere organische Polyole enthält, die aus Glycerin, Sorbit, Maltit, Xylit und Mischungen davon ausgewählt sind.

7. Verwendung nach irgendeinem vorhergehenden Anspruch, wobei der Anteil des Carrageens von 0,1 bis 3 Gew.-% der Gesamtzusammensetzung beträgt.

## Revendications

1. Utilisation de carraghénane dans une tablette pour le soin oral pour promouvoir la durée de rupture de la tablette dans la bouche.

2. Utilisation selon la revendication 1, dans laquelle la tablette est une tablette comprimée solide pour le soin oral, la composition de la tablette comprenant un mélange de carbonate de calcium et de carraghénane, dans laquelle le rapport massique du carraghénane au carbonate de calcium est de 1:20 à 1:00.

3. Utilisation selon la revendication 1 ou la revendication 2, dans laquelle le carraghénane est du carraghénane kappa.

4. Utilisation selon la revendication 2 ou la revendication 3, dans laquelle le carbonate de calcium présente une taille de particule d(50) de 0,1 à 30 microns, de préférence de 0,1 à 15 microns, encore mieux de 2 à 10 microns comme mesurée par un Mastersizer Malvern.

5. Utilisation selon l'une quelconque des revendications précédentes, dans laquelle la tablette comprend de plus un agent moussant choisi dans le groupe consistant en tensioactifs anhydres choisis parmi le laurylsulfate de sodium, laurylsulfoacétate de sodium, cocamidopropylbétaïne, alpha-oléfinesulfonate de sodium, sulfosuccinate de dioctylsodium, dodécylbenzènesulfonate de sodium et des mélanges de ceux-ci.

6. Utilisation selon l'une quelconque des revendications précédentes, dans laquelle la composition comprend également un ou plusieurs polyols organiques choisis parmi le glycérol, sorbitol, maltitol, xylitol et mélanges de ceux-ci.

7. Utilisation selon l'une quelconque des revendications précédentes, dans laquelle la teneur en carraghénane est de 0,1 à 3 % en masse de la composition totale.
